# EUROPEAN PATENT SPECIFICATION

(11) **EP 2 419 080 B1**
(45) Date of publication and mention of the grant of the patent: **25.07.2018**
(21) Application number: 10715424.7
(22) Date of filing: 15.04.2010
(51) Int. Cl.: A61K 8/04, A61K 8/85, A61Q 19/10, A61K 8/02

(54) **MICRONIZED POLYMER POWDER COSMETIC COMPOSITION**
KOSMETISCHE ZUSAMMENSETZUNG ENTHALTEND EIN MIKRONISIERTES POLYMERPULVER
COMPOSITION COSMÉTIQUE RENFERMANT UNE POUDRE DE POLYMÈRE MICRONISÉE

(30) Priority: 17.04.2009 EP 09290289
(43) Date of publication of application: 22.02.2012
(73) Proprietor: Coatings Foreign IP Co. LLC, Wilmington, DE 19801 (US)
(72) Inventor: MAGNIN, Olivier, CH-1005 Lausanne (CH); GHERARDI, Carole, F-01710 Thoiry (FR); RASPAIL, Vincent, F-01210 Ferney-Voltaire (FR); GRANJOU, Ludovic, CH-1800 Vevey (CH)
(74) Representative: LKGLOBAL Lorenz & Kopf PartG mbB Patentanwälte
(86) International application number: PCT/US2010/031249
(87) International publication number: WO 2010/121032

(56) References cited:
- EP-A1- 2 241 307
- YOKOI T: "Microfine polyester powder useful for cosmetics - obtd. by jetting polyester particles against target at high speed using compressed gas", WPI / THOMSON,, vol. 1987, no. 40, 1 September 1987 (1987-09-01), XP002547478,
- DATABASE CAPLUS, [Online] 21 March 2008 (2008-03-21), IMANAKA HIROSHI ET AL: "Cosmetic makeup containing polyethylene terephthalate powder", XP002547477, retrieved from CAPLUS Database accession no. 2008-348566
- HOTSUTA Y ET AL: "Fine polyester particles dispersion - useful as seed particles for prepn of crosslinked particles useful in powder paint, toner, cosmetics, pharmaceutical carrier etc", WPI / THOMSON,, vol. 1994, no. 42, 20 September 1994 (1994-09-20), XP002547479,

## Description

### Field of the Invention

The invention is directed to a micronized polymer powder composition made from at least one thermoplastic polymer for the application in personal care and cosmetics, to a process of preparation of the micronized polymer powder composition and to a cosmetic composition thereof.

### Description of Prior Art

Micronized polymer powders are widely used as additives and compounds in the cosmetic industry. Micronized polymer powders are used due to their exfoliating properties. Exfoliating is the meaning of scrubbing the skin surface with solid particles which, while moving on the skin surface, will remove the dead cells and the impurities from it. Products based on micronized polymer powders can be used to clean the skin surface providing deep purification, anti-aging properties as well as to provide a smoother, softer and brighter skin surface.

In general, micronized polymer powders are made from polymers based on crude oil. For example, polyethylene beads can provide good exfoliation properties and can avoid micro cuts that can occur on the skin when the exfoliation is made with natural material like nut shell powder. The use of petrochemicals is critical because of decreased oil resources, increased processing costs, increased energy consumption and production of carbon dioxide harmful to the environment. Also, public concern about the health hazards arising from healthcare and cosmetic products which are prepared from crude oil.

An alternative is to use natural based (bio-based) exfoliating powders like common natural based apricot kernel powders, nut or nut shell powders, compositions based on sea salt, sugar, rice powder, silica and fruit seeds. Products based on such powders are known to be coarser, and they can cause micro-cuts and can irritate the skin surface.

Therefore, there is a need for improved micronized powders for the application in personal care and cosmetics based on natural resources avoiding petrochemical resources.

### Summary of the Invention

The present invention provides a micronized polymer powder composition for the use in personal care and cosmetics, the micronized polymer powder composition comprising at least one thermoplastic polyester based on at least one diol and at least one dicarboxylic acid selected from the group consisting of cyclic and branched aliphatic dicarboxylic acids having 4-12 carbon atoms and aromatic dicarboxylic acids having 8-12 carbon atoms, wherein the particles of the micronized polymer powder composition having an average particle size in the range of 200 to 800 µm.

The present invention provides also a cosmetic composition comprising the at least one micronized polymer powder composition according to the invention.

The micronized polymer powder composition according to the invention provides a high-performed gentle exfoliation property of skin care and cosmetic compositions without creating micro cuts and irritation of the skin surface while still providing the range of desired care properties like deep purification, anti-aging properties as well as smooth, soft and bright skin surface. The powder composition according to the invention can be prepared with an average particle size well-defined for cosmetic end-use applications.

### Detailed Description of the Invention

The features and advantages of the present invention will be more readily understood, by those of ordinary skill in the art, from reading the following detailed description. It is to be appreciated those certain features of the invention, which are, for clarity, described above and below in the context of separate embodiments, may also be provided in combination in a single embodiment. Conversely, various features of the invention that are, for brevity, described in the context of a single embodiment, may also be provided separately or in any subcombination. In addition, references in the singular may also include the plural (for example, "a" and "an" may refer to one, or one or more) unless the context specifically states otherwise.

All patents, patent applications and publications referred to herein are incorporated by reference in their entirety.

The micronized polymer powder composition according to the invention for the use in personal care and cosmetics is a powder composition comprising at least one thermoplastic polyester based on at least one diol and at least one dicarboxylic acid selected from the group consisting of cyclic and branched aliphatic dicarboxylic acids having 4-12 carbon atoms and aromatic dicarboxylic acids having 8-12 carbon atoms.

Examples of such polyesters are polyalkylene terephthalate, for example trimethylene terephtalate, or polyesters produced by polymerization between at least one diol and at least one carboxylic acid such butanedioic acid, pentanedioic acid, hexanedioic acid, dodecanedioic acid, and 1,4-cyclo-hexanedicarboxylic acid, terephthalic acid, isophthalic acid and 2,6-naphthalenedicarboxylic acid.

The at least one diol is selected from the group consisting of ethanediol, propanediol, 1,4-butanediol, 3-methyl-1,5-pentanediol, 2,2-dimethyl-1,3-propanediol, 2-methyl-1,3-propanediol, and 1 ,4-cyclohexanediol. The diol is at least one bio-based diol.

The term "bio-based diol" means a diol that is derived from agricultural corn by processes known in the art. In principle, such processes comprise the steps of harvesting the corn, getting sugar from the corn and transferring the sugar into the alcohol (diol) by fermentation.

The thermoplastic polyester according to the invention can be prepared by processes known in the art. According to the invention the polyester is prepared using the at least one bio-based diol.

The content of the at least one bio-based diol in the at least one thermoplastic polyester can very in a range of 10 to 100 wt%, preferred 20 to 40 wt%, based on the used diol component in the polyester.

The micronized polymer powder composition according to the invention may also contain further components. These components can be non-polymer components such as colorants, for example pigments, for example in a range of 0 to 10 wt% based on the micronized powder composition of the invention. Examples of pigments are titanium dioxide pigments.

Further components can be further polymers different from the at least one thermoplastic polyester, for example in a range of 0 to 30 wt% based on the micronized powder of the invention.

The micronized polymer powder composition according to the invention may be prepared by first mixing the components of the powder composition and then micronizing the mixture. This can be done by processes known in the art.

For example, the powder composition of the invention is first prepared as pellets and/or granulates by combining the components of the composition by using melt-mixing techniques known in the art. For example, the polymer components and non-polymer components of the composition may be added to a melt mixer known in the art, for example, an extruder; a blender, kneader, roll mixer, or other kind of mixers, and then melt-mixed. When adding the components of the composition of the invention in a stepwise fashion, a part of the components of the composition are first added and melt-mixed with the remaining components of the composition being subsequently added and further melt-mixed until a well-mixed composition is obtained. Then, the compounded pellets and/or granulates so-obtained are sized to a fine powder by using methods known in the art of micronizing. For example, the extruded material is granulated and then ground at room temperature or at low temperature to a fine powder, which can be classified to the desired particle size. For example, the compounded pellets are cooled at low temperatures, preferably between -120 to -200°C and more preferably between -140 to -180°C. The brittle extra-cold granules are then fed into a grinder that reduces the granules to fine particles by using, for example, an impact or attrition milling process known by a person skilled in the art. The milling technique is selected by the person skilled in the art in function of the ductility of the compounded pellet to grind and the final particle size distribution targeted. For obtaining desired size, the grinding step is associated with a sieving step for eliminating the large particles and the fine size particles.

The particles of the micronized polymer powder composition have an average particle size in the range of 200 to 800 µm. The average particle size can be selected in a range desired for a specific application in the personal care and cosmetics. Smaller particles can be used for volumizing effects, soft focus effect in skin care or in decorative cosmetics (make-up), for skin sebum absorption in skin care or decorative cosmetics (mattifying effect), for easing the process of decorative powders compaction, to improve the decorative powders properties (modulate pay-off, transparency, handling, texture and touch, long lasting effect on skin etc), improving the touch feeling (soft touch) and the texture of skin care and decorative cosmetics, for example in a range of 0.1 to 100 µm, preferred in a range of 1 to 20 µm.

For example, the particles of the micronized polymer powder composition of the invention has a particle size (D90 value) less, than or equal to 300 µm, more preferably between about 200 and about 280 µm. The D90 value corresponds to a particle size below which 90 wt-% of the particles lie, wherein the particle size analysis is done by a laser diffraction method and meets the standards set forth in ISO 3310-1. Measurements can be done on a Malvern Mastersizer 2000.

The micronized polymer powder composition can be used as exfoliating component in personal care and cosmetic compositions in amounts in a range of, for example, 1 to 10 wt%, preferably 2 to 5 wt%, based on the total cosmetic composition. The amounts can be adapted with the specific personal care and cosmetic end-use application, for example, 2 to 5 wt% for face or sensitive body parts, 5 to 10 wt% for body. Therefore, this invention also refers to a cosmetic composition comprising the at least one micronized polymer powder composition described above.

The present invention is further defined in the following Examples. It should be understood that these Examples are given by way of illustration only.

### Examples

### Example 1

### Test of cosmetic products containing exfoliating component of the invention and comparison product

A cleansing face gel product A was containing 3 wt% of polyethylene beads as exfoliating component, a cleansing face gel product product B was containing 3 wt% of the polymer powder compositionof the invention as exfoliating component. The formulations of these products were identical, except the kind of the exfoliating component. The exfoliating component of products A and B had the same particle size distribution of < 315 µm.
An panel test was performed wherein the panellists have been using the 2 cleansing face gel products everyday during 3 consecutive days for each product. The product distribution to the panellist was randomised. Half the panel started to test the product A and the other half started to test the product B. After 3 days they changed and tested the other (A or B) product.

### Example 2

### Test Result: Exfoliating Efficiency

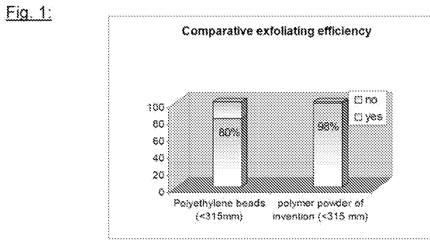

The test results show that the product including the polymer powder composition of the invention provides a superior exfoliating action versus the product including the polyethylene component, see Fig. 1.

## Claims

1. A micronized polymer powder composition for the use in personal care and cosmetics comprising at least one thermoplastic polyester based on at least one diol and at least one dicarboxylic acid selected from the group consisting of cyclic and branched aliphatic dicarboxylic acids having 4-12 carbon atoms and aromatic dicarboxylic acids having 8-12 carbon atoms, wherein the particles of the micronized polymer powder composition have an average particle size in the range of 200 to 800 µm,
whereby the thermoplastic polyester is based on at least one diol selected from the group consisting of ethanediol, propanediol, 1,4-butanediol, 3-methyl-1,5-pentanediol, 2,2-dimethyl-1,3-propanediol, 2-methyl-1,3-propanediol, and 1,4-cyclohexanediol and at least one dicarboxylic acid selected from the group consisting of butanedioic acid, pentanedioic acid, hexanedioic acid, dodecanedioic acid, 1,4-cyclo-hexanedicarboxylic acid, terephthalic acid, isophthalic acid and 2,6-naphthalenedicarboxylic acid, wherein the diol is at least one bio-based diol.

2. The composition according to claim 1 wherein the content of the at least one bio-based diol in the at least one thermoplastic polyester is in a range of 20 to 40 wt%, based on the used diol component in the polyester.

3. The composition according to claim 1 or 2comprising
A) the at least one thermoplastic polyester based on at least one diol and at least one dicarboxylic acid selected from the group consisting of cyclic and branched aliphatic dicarboxylic acids having 4-12 carbon atoms and aromatic dicarboxylic acids having 8-12 carbon atoms,
B) optionally, preferably 0.1 to 10 wt%, of at least one non-polymer component, and
C) optionally, preferably 0.1 to 30 wt%, of at least one polymer different from the the at least one thermoplastic polyester A),
the wt% amounts based on the total weight of the composition (A) to (C).

4. A process of preparation of the micronized polymer powder composition according to claims 1 to 3 comprising the steps
a) mixing the components of the powder composition, and
b) micronizing the mixture.

5. A cosmetic composition comprising the micronized polymer powder composition of claims 1 to 3.

6. The cosmetic composition according to claim 5 wherein the content of the micronized polymer powder composition is in a range of 1 to 10 wt% based on the total weight of the cosmetic composition.

## Patentansprüche

1. Mikronisierte Polymerpulverzusammensetzung zur Verwendung in der Körperpflege und Kosmetik, umfassend zumindest einen thermoplastischen Polyester, der auf zumindest einem Diol und zumindest einer Dicarbonsäure, die aus der Gruppe ausgewählt ist, bestehend aus zyklischen und verzweigten aliphatischen Dicarbonsäuren, die 4-12 Kohlenstoffatome aufweisen, und aromatischen Dicarbonsäuren, die 8-12 Kohlenstoffatome aufweisen, basiert, worin die Teilchen der mikronisierten Polymerpulverzusammensetzung eine durchschnittliche Teilchengröße im Bereich von 200 bis 800 µm aufweisen,
wobei der thermoplastische Polyester auf zumindest einem Diol, das aus der Gruppe ausgewählt ist, bestehend aus Ethandiol, Propandiol, 1,4-Butandiol, 3-Methyl-1,5-pentandiol, 2,2-Dimethyl-1,3-propandiol, 2-Methyl-1,3-propandiol und 1,4-Cyclohexandiol, und zumindest einer Dicarbonsäure, die aus der Gruppe ausgewählt ist,
bestehend aus Butandisäure, Pentandisäure, Hexandisäure, Dodecandisäure, 1,4-Cyclohexandicarbonsäure, Terephthalsäure, Isophthalsäure und 2,6-Naphthalendicarbonsäure, basiert, wobei das Diol zumindest ein bio-basiertes Diol ist.

2. Zusammensetzung gemäß Anspruch 1, wobei der Gehalt des zumindest einen biobasierten Diols in dem zumindest einem Polyester in einem Bereich von 20 bis 40 Gew.-%, basierend auf dem verwendeten Diolbestandteil in dem Polyester, liegt.

3. Zusammensetzung gemäß Anspruch 1 oder 2, umfassend
A) den zumindest einen thermoplastischen Polyester, der auf zumindest einem Diol und zumindest einer Dicarbonsäure, die aus der Gruppe ausgewählt ist, bestehend aus zyklischen und verzweigten aliphatischen Dicarbonsäuren, die 4-12 Kohlenstoffatome aufweisen, und aromatischen Dicarbonsäuren, die 8-12 Kohlenstoffatome aufweisen, basiert,
B) wahlweise, bevorzugt 0,1 bis 10 Gew.-%, von zumindest einem Nicht-Polymerbestandteil, und
C) wahlweise, bevorzugt 0,1 bis 30 Gew.-%, von zumindest einem Polymer, das von dem zumindest einem thermoplastischen Polyester A) verschieden ist,
wobei die Gew.-%-Mengen auf dem Gesamtgewicht der Zusammensetzung (A) bis (C) basieren.

4. Verfahren zur Herstellung einer mikronisierten Polymerpulverzusammensetzung gemäß einem der Ansprüche 1 bis 3, umfassend die Schritte
a) Mischen der Bestandteile der Pulverzusammensetzung, und
b) Mikronisieren der Mischung

5. Kosmetische Zusammensetzung, umfassend die mikronisierte Polymerpulverzusammensetzung nach den Ansprüchen 1 bis 3.

6. Kosmetische Zusammensetzung gemäß Anspruch 5, worin der Gehalt der mikronisierten Polymerpulverzusammensetzung in einem Bereich von 1 bis 10 Gew.-%, basierend auf dem Gesamtgewicht der kosmetischen Zusammensetzung, liegt.

## Revendications

1. Composition renfermant une poudre de polymère micronisée destinée à l'utilisation dans le cadre des soins personnels et des cosmétiques comprenant au moins un polyester thermoplastique sur au moins un diol et au moins un acide dicarboxylique provenant du groupe constitué des acides dicarboxyliques aliphatiques cycliques ou ramifiés possédant de 4 à 12 atomes de carbone et des acides dicarboxyliques aromatiques possédant de huit à 12 atomes de carbone, dans laquelle les particules de la composition renfermant une poudre de polymère micromisée possèdent une particule de taille moyenne dans la gamme allant de 200 à 800 µm, selon lequel le polyester thermoplastique est basé sur au moins un diol choisi parmi le groupe constitué de l'éthanediol, du propanediol, du 1,4-butanediol, du 3-méthyl-1,5-pentadiol, du 2,2-diméthyl-1,3-propanediol, du 2-méthyl-1,3-propanediol et du 1,4-cyclohexanediol et d'au moins un acide dicarboxylique choisi parmi le groupe constitué de l'acide butanedioïque, de l'acide pentanedioïque, de l'acide hexanedioïque, de l'acide dodécanedioïque, de l'acide 1,4-cyclo-hexanedicarboxylique, de l'acide téréphtalique, de l'acide isophtalique et de l'acide 2,6-naphtalènedicarboxylique, dans lequel le diol et au moins un diol d'origine biologique.

2. Composition selon la revendication 1, dans laquelle la teneur de l'au moins un diol d'origine biologique dans l'au moins un polyester thermoplastique est comprise dans la gamme allant de 20 à 40 % en poids, sur la base du composant diol utilisé dans le polyester.

3. Composition selon la revendication 1 ou la revendication 2, comprenant
A) l'au moins un polyester thermoplastique sur la base de l'au moins un diol et l'au moins un acide dicarboxylique choisi parmi le groupe constitué des acides dicarboxyliques aliphatiques cycliques et ramifiés possédant de 4 à 12 atomes de carbone et des acides dicarboxyliques aromatiques possédant de 8 à 12 atomes de carbone,
B) éventuellement, de préférence entre 0,1 et 10 % en poids d'au moins un composant non polymérique, et
C) éventuellement, de préférence entre 0,1 et 30 % en poids, d'au moins un polymère différent de l'au moins un polyester thermoplastique A),
les teneurs de pourcentage massique sont basées sur le poids total de la composition (A) à (C).

4. Procédé de préparation de la composition renfermant une poudre de polymère micronisée selon l'une quelconque des revendications 1 à 3, comprenant les étapes consistant à
a) mélanger les composants de la composition de poudre, et
b) microniser le mélange.

5. Composition cosmétique comprenant la composition renfermant la poudre de polymère micronisée selon l'une quelconque des revendications 1 à 3.

6. Composition cosmétique selon la revendication 5, dans laquelle la teneur de la composition renfermant la poudre de polymère micronisée est comprise dans la gamme allant de 1 à 10 % en poids sur la base du poids total de la composition cosmétique.
